# EUROPEAN PATENT APPLICATION

(11) **EP 1 829 495 A1**
(43) Date of publication of application: **05.09.2007**
(21) Application number: 05819459.8
(22) Date of filing: 21.12.2005
(51) Int. Cl.: A61B 19/00, A61B 1/00, A61B 1/12

(54) **MEDICAL DEVICE AND PROCESSING COUNTER FOR MEDICAL DEVICE**

(30) Priority: 24.12.2004 JP 2004374971
(71) Applicant: Olympus Corporation, Tokyo 151-0072 (JP)
(72) Inventor: HATORI, Tsuruo, c/o Olympus Corporation, Tokyo 151-0072 (JP)
(74) Representative: von Hellfeld, Axel
(86) International application number: PCT/JP2005/023492
(87) International publication number: WO 2006/068179

(57) **Abstract**

The present invention provides medical equipment and medical equipment treatment execution counter unit which presents the number of sterilization process executed to the medical equipment. The medical equipment and the medical equipment treatment execution counter unit include a sealed chamber which has an elastic film that exhibits elasticity at least partially and a sealed space isolated from the outside by the elastic film, and counter means for counting the number of deformation of the elastic film.

## Description

### Technical Field

The present invention relates to medical equipment and medical equipment treatment execution counter unit capable of detecting and storing the number of the treatment executed to the medical equipment, for example, washing, disinfection and sterilization.

### Background Art

The medical equipment to be repeatedly used has been conventionally subjected to such treatment as washing, disinfection and sterilization after it is used for the diagnosis and the therapy as the general practice. In the treatment executed to the medical equipment, washing, disinfection and sterilization (hereinafter simply referred to as sterilization), the treatment with the chemical material, heating, and the physical treatment may be performed to eliminate the waste or to sterilize the microorganism. However, as the sterilization exerts the chemical, thermal and physical stress to the medical equipment, the latent damage of the medical equipment may be intensified by the respective sterilization process.

Therefore, the user of the medical equipment is required to execute the sterilization with respect to the medical equipment within the allowable number of times, and to perform the maintenance/repair recommended by the manufacturer for the medical equipment before it becomes out of work for the purpose of using the medical equipment safely for the elongated service life. For this, the user of the medical equipment has to obtain the number of executing the sterilization performed to the respective medical equipment objectively.

Japanese Unexamined Patent Application Publication No. 11-56746 discloses the endoscope as a medical equipment which allows the user to obtain the number of executing the sterilization to the medical equipment.

The endoscope as described in the first and the second embodiments referring to Figs. 1 to 4 in Japanese Unexamined Patent Application Publication No. 11-56746 is formed of the detection means as a compound sensor provided at a connector of the endoscope for measuring temperatures, pressures and pHs, the determination means for determining the type of the sterilization process based on the information derived from the detection means and the preliminarily stored information, the count means which counts the number of executing the sterilization of the type determined by the determination means to the endoscope such that the user is informed of the counted number, and power means provided with a battery for supplying power for operating the detection means, the determination means and the count means. The power means is provided with an operation power supply connector so as to be connected to the external power supply unit.

Based on the number of executing the sterilization counted by the count means of the endoscope, the user obtains the number of executing the sterilization to the endoscope such that the timing required for performing the maintenance/repair recommended by the manufacturer is determined.

In the endoscope as described in the third and the fourth embodiments referring to Figs. 5 and 6 in Japanese Unexamined Patent Application Publication No. 11-56746, the endoscope connector is provided with an alarm index formed of a member which is eroded when it is immersed into the chemical sterilizing solution, or an alarm index fixed with the adhesive agent, the adhesive force of which is weakened when it is immersed into the chemical sterilizing solution.

Based on the erosion state of the alarm index caused by the chemical sterilizing solution used in the sterilization process, or the state whether or not the alarm index peels off owing to weakening of the adhesive force of the adhesive agent caused by the chemical sterilizing solution used in the sterilization process, the user recognizes the service limit, and determines the time to execute the maintenance/repair recommended by the manufacturer.

The endoscope proposed in the first and the second embodiments in Japanese Unexamined Patent Application Publication No. 11-56746 includes the detection means as the compound sensor for detecting pressures, temperatures and pHs such that the number of executing the sterilization under the sterilizing treatment environment is counted. The endoscope is required to allow the power means to constantly supply the operation power for operating not only the detection means but also the determination means and the count means. The battery consumption undergoes at substantially the higher rate when the power for operating the detection means, the determination means and the count means is constantly supplied from the battery. The user is required to monitor the state of charge of the battery on a constant basis. The operation power supply connector has been practically used with respect to the function specific to the endoscope with the connector for coupling the endoscope to the external units such as the light source device and the video processor. Accordingly, the versatility applied to the medical equipment without the connector for connecting the external units is insufficient.

The endoscope proposed in the third and the fourth embodiments in Japanese Unexamined Patent Application Publication No. 11-56746 is structured to inform the service limit of the medical equipment to the user based on the alarm index which is chemically eroded by the chemical sterilizing solution or the alarm index which peels off owing to the weakened adhesive force. The user is kept unknown with respect to the specific number of executing the sterilizing process until the alarm index is eroded to disappear or the alarm index peels off owing to weakening of the adhesive force.

It is an object of the present invention to provide a medical equipment and a medical equipment treatment execution counter unit for constantly providing the number of executing the sterilization.

### Disclosure of Invention

### Means for Solving the Problem

The present invention provides a medical equipment provided with a count unit for counting a number of executing a treatment including at least one of washing, disinfection and sterilization to the medical equipment. The count unit includes a sealed chamber which has an elastic film that exhibits elasticity at least partially and a sealed space isolated from the outside by the elastic film, and count means for counting the number of deformation of the elastic film.

The sealed chamber of the medical equipment according to the present invention is formed in a space of a housing contained therein having a vent hole formed to communicate the space with the outside.

The housing of the medical equipment according to the present invention is provided to a part of an exterior member of the medical equipment or formed as a part of the exterior member.

A check valve for sealing the vent hole is provided when a pressure of the space in the housing of the medical equipment according to the present invention becomes negative with respect to the pressure outside the housing.

The count unit of the medical equipment according to the present invention further includes a display unit which displays a value counted by the count means.

The count means of the medical equipment according to the present invention includes a first electric contact disposed on the elastic film, a second electric contact disposed on a position opposite the first electric contact, and a counter unit which detects a power application state where the first electric contact and the second electric contact are brought into contact by deformation of the elastic film, and counts to store the number of the power application.

The count means of the medical equipment according to the present invention includes a push button mechanically depressed by the deformed elastic film, and a counter device which counts up a number of depression of the push button.

A medical equipment treatment execution counter unit according to the present invention counts a number of executing a treatment including at least one of washing, disinfection and sterilization to the medical equipment, and includes a sealed chamber which has an elastic film that exhibits elasticity at least partially, and a sealed space isolated from the outside by the elastic film, and count means for counting the number of deformation of the elastic film.

### Brief Description of the Drawings

Fig. 1 is an outline view of an endoscope as a medical equipment according to the present invention.
Fig. 2 is a sectional view showing a concept of the medical equipment treatment execution counter unit according to a first embodiment of the present invention.
Fig. 3 is a timing chart showing the pressure change in the chamber under the sterilization process by the autoclave sterilizing unit.
Fig. 4 is a sectional view showing a concept of the medical equipment treatment execution counter unit according to a second embodiment of the present invention.
Fig. 5 is a sectional view showing a concept of the medical equipment treatment execution counter unit according to a third embodiment of the present invention.

### Best Mode for Carrying Out the Invention

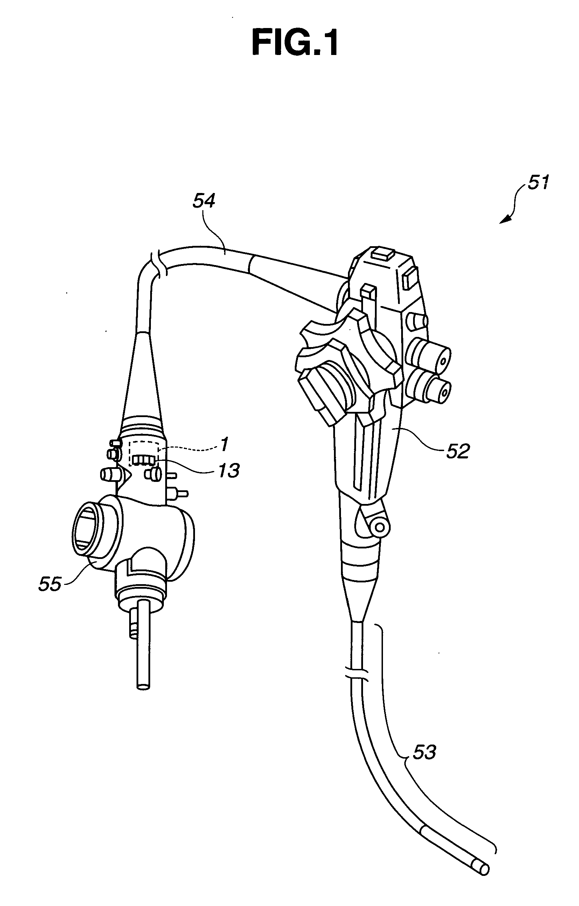

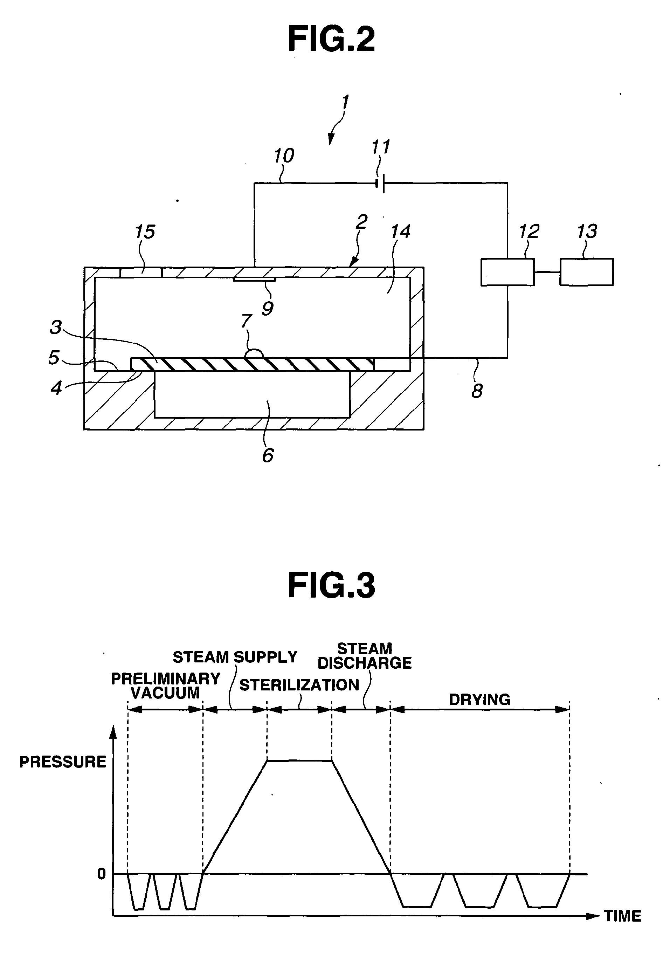

An embodiment of the present invention will be described in detail referring to the drawings. A medical equipment according to the first embodiment of the present invention will be described referring to Figs. 1, 2 and 3. Fig. 1 is an outline view of an endoscope as a medical equipment according to the present invention. Fig. 2 is a sectional view showing a concept of the medical equipment treatment execution counter unit according to the present invention. Fig. 3 is a timing chart showing the pressure change in the chamber under the sterilization process by the autoclave sterilizing device.

Referring to Fig. 1, an endoscope 51 as the medical equipment according to the present invention includes an insertion portion 53, an operation portion 52, a joint cord 54, and a connector portion 55. The insertion portion 53 is flexibly formed into a thin and long shape. The operation portion 52 is connected to a proximal end of the insertion portion 53. The joint cord 54 which exhibits flexibility extends from the side portion of the operation portion 52. The connector portion 55 is provided to the end of the joint cord 54, and connected to the light source device and the video processor which are not shown. A medical equipment treatment execution counter unit 1 is provided to the connector portion 55 of the endoscope 51. A display unit 13 of the medical equipment treatment execution counter unit 1 is disposed on the exterior of the connector portion 55.

Referring to Fig. 2, in the medical equipment treatment execution counter unit 1 according to the first embodiment of the present invention, a housing 2 including substantially a cube-like space 14 is formed in a part of the medical equipment (not shown). The housing 2 may be disposed on the scope connector, that is, the connector portion in the case where the endoscope is used as the medical equipment, and may be disposed on a grasping portion grasped by the operator for operations in the case where the forceps for various medical treatments is used as the medical equipment. A vent hole 15 is formed in the housing 2 for ventilating the ambient atmosphere around the housing 2 into the space 14. The space 14 in the housing 2 is kept at the same pressure state as the ambient pressure of the housing 2 through the vent hole 15, that is, it is normally kept at substantially the same pressure as the atmospheric pressure. A vent hole for communicating outside the medical equipment with the periphery of the housing 2 is formed in the medical equipment such that the outside of the medical equipment and the space 14 in the housing 2 are kept under the same pressure state. The vent hole 15 may be formed to directly communicate the outside of the medical equipment with the space 14 in the housing 2. The housing 2 may be formed as a part of the exterior member of the medical equipment. In this case, the outside of the medical equipment and the space 14 in the housing 2 may only be communicated via the vent hole 15.

A gas chamber 6 as a sealed chamber separated by an elastic film 3 so as to be in the sealed state is formed in the space 14 of the housing 2. The gas chamber 6 is formed as substantially a cube-like shaped which is smaller than the space 14. A stepped portion 5 is formed at a boundary between the gas chamber 6 and the space 14. A side edge 4 of the elastic film 3 which is air tightly separates the gas chamber 6 from the space 14 is fixed to the stepped portion 5 with the adhesive agent.

The elastic film 5 is formed of an elastic member such as a rubber material. The gas chamber 6 filled with gas like air is sealed with the elastic film 5 at substantially the same pressure as the atmospheric pressure. When the pressure within the space 14 becomes negative with respect to the atmospheric pressure, the pressure difference between the gas chamber 6 and the space 14 deforms the elastic film 3 toward the space 14 such that the gas chamber 6 expands.

A first electric contact 7 is formed at a substantial center (center on the upper surface shown in the drawing) on the surface of the elastic film 3 to face the space 14. The first electric contact 7 is connected to one end of a first signal line 8 extending outward of the housing 2.

A second electric contact 9 is formed on the inner surface of the housing 2 opposite the first electric contact 7 formed on the elastic film 3. The second electric contact 9 is formed at the position which is brought into contact with the first electric contact 7 when the gas chamber 6 expands to deform the elastic film 3. The second electric contact 9 is connected to one end of a second signal line 10 extending outward of the housing 2.

A battery 11 and a counter unit 12 are connected in series between the first signal line 8 and the other end of the second signal line 10. When the first electric contact 7 is electrically coupled with the second electric contact 9 to receive power from the battery 11, the counter unit 12 detects the power application, and counts the number of the power application so as to be stored. The counter unit 12 is structured by the known art using the flash memory or the like. Specifically, the first and the second electric contacts 7, 9, the first and the second signal lines 8, 10, the battery 11 and the counter unit 12 form the count means which counts deformation of the elastic film 3.

The display unit 13 is connected to the counter unit 12. The display unit 13 is structured by the known counter display means intended to display the number of power application which has been detected, counted and stored by the counter unit 12 for the user. The medical equipment treatment execution counter unit 1 does not have to include the display unit 13. Alternatively, the display unit 13 may be connected to the counter unit 12 in response to the request of the user who requires to know the number of the power application.

The housing 2, the first and the second signal lines 8, 10, the battery 11 and the counter unit 12 are formed to be embedded inside the medical equipment. The display unit 13 is provided to the outer surface of the medical equipment. In the case where the medical equipment manufacturer requires to obtain the number of performed sterilization for monitoring how the medical equipment has been used for the medical treatment, the display unit 13 may be formed inside the medical equipment so as to obtain the number of sterilization by disassembling the equipment which has been used in the market.

The pressure of the space 14 in the housing 2 decreases accompanied with reduction in the atmospheric pressure around the housing 2, and increases accompanied with the increase in the atmospheric pressure through the vent hole 15.

An example of the sterilization process using the autoclave sterilizing unit for the medical equipment will be described referring to Fig. 3. The horizontal axis of the timing chart shown in Fig. 3 represents the time, and the longitudinal axis represents the pressure in the chamber of the sterilizing unit.

The sterilization process performed through the autoclave sterilizing unit includes preliminary vacuum step, steam supply step, sterilization step, steam discharge step and drying step. In the preliminary vacuum step, the vacuuming of the chamber in the autoclave sterilizing unit is performed three times. Upon each vacuuming, high temperature/pressure steam is introduced into the chamber such that air in the chamber is substituted by the steam. In the steam supply step followed by the preliminary vacuum step, the steam at the positive pressure is supplied into the chamber. In the sterilization step, the medical equipment as the subject to be sterilized in the chamber is exposed to the steam at a predetermined set temperature for a predetermined time period. In the steam discharge step followed by the sterilization step, the steam is discharged from the chamber. After discharging the steam from the chamber in the steam discharge step, the drying step is performed. Unlike the preliminary vacuum step, in the drying step, at every vacuuming in the chamber to be performed three times, air is introduced into the chamber to dry the subject to be sterilized which has been wet by the steam. In the drying step, vacuuming is performed to boil water adhered to the subject to be sterilized so as to be vaporized out of the chamber.

In the sterilization process through the autoclave sterilizing unit, vacuuming is performed in total of six times from the preliminary vacuum step to the drying step as shown in Fig. 3. The number of performing the vacuuming is preliminarily programmed by the autoclave unit. The programmed number is not changed by the respective sterilizations. As the number of vacuuming in the chamber is detected and recorded by the medical equipment, it is possible for the user to calculate the number of performing the autoclave sterilization to the medical equipment by dividing the total number by six as the number of vacuuming in a single sterilization process as a unit.

The operation of the sterilization process executed to the medical equipment which includes the medical equipment treatment execution counter unit 1 through the autoclave sterilizing unit will be described.

When the pressure in the chamber of the autoclave sterilizing unit becomes negative, the pressure of the space 14 in the housing 2 becomes negative to be the same as the ambient pressure in the chamber via the vent hole 15. When the pressure of the space 14 in the housing 2 becomes negative, the pressure difference with respect to the gas chamber 6 occurs (pressure in the gas chamber 6 > pressure in the space 14). The pressure difference between the gas chamber 6 and the space 14 deforms the elastic film 3 to expand toward the space 14 at the low pressure side (upward direction in the drawing). When the elastic film 3 deforms to expand, the first electric contact 7 is moved upward as shown in the drawing to be in contact with the second electric contact 9.

When the first electric contact 7 contacts the second electric contact 9, that is, they become ON state, the circuit including the battery 11 and the counter unit 12 is closed such that power is supplied from the battery 11 to the counter unit 12 via the first and the second electric contacts 7 and 9. The counter unit 12 detects to count the power application caused by the contact between the first and the second electric contacts 7 and 9 to become ON state.

When the first vacuuming is finished, the ambient pressure within the chamber becomes the atmospheric pressure, and the pressure of the space 14 in the housing 2 also becomes the atmospheric pressure. The pressure of the gas chamber 6 becomes substantially the same as that of the space 14. When the pressure of the gas chamber 6 becomes substantially the same as that of the space 14, the elastic film 3 returns to the original state before the vacuuming under the elastic force. The first electric contact 7 moves away from the second electric contact 9 into OFF state. In OFF state, the power application from the battery 11 is interrupted.

In the single sterilization process, the vacuuming is performed in the chamber in total of six times from the preliminary vacuum step to the drying step. The first and the second electric contacts 7 and 9 become ON state six times. Accordingly, the counter unit 12 records the number of the power application as six in the single sterilization process. That is, atmosphere around the medical equipment is vacuumed six times in total. The number of vacuuming of the atmosphere in the medical equipment which has been cumulatively recorded by the counter unit 12 is displayed on the display unit 13.

The medical equipment treatment execution counter unit 1 according to the first embodiment provides the following effects.

The user is capable of obtaining the number of executing sterilization process to the medical equipment by dividing the number of vacuuming of the atmosphere around the medical equipment displayed on the display unit 13 by the number of vacuuming performed in the single sterilization. For example, if the number of vacuuming of the atmosphere around the medical equipment displayed on the display unit 13 is 60, the user divides the number sixty by six to obtain the specific number of sterilization to the subject medical equipment, that is, 10 times.

The power is supplied to the first and the second electric contacts 7 and 9 only in the state where the chamber pressure is negative owing to the vacuuming. As the power applied from the battery 11 is used only to drive the counter unit 12, the level of the required power is low. The draining of the battery 11, thus, may be kept low, resulting in elongated period of life. The user does not have to constantly monitor the state of charge of the battery.

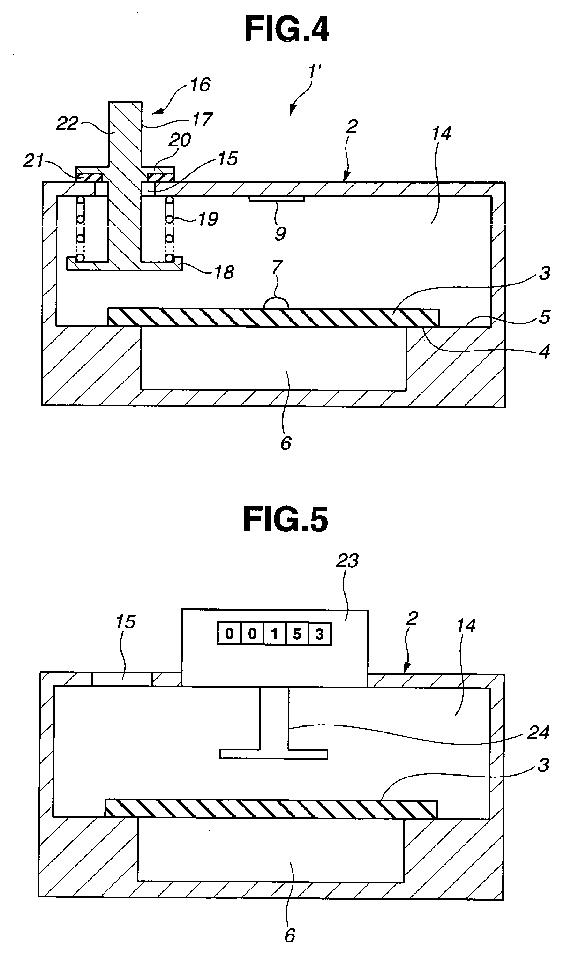

A medical equipment treatment execution counter unit according to the second embodiment of the present invention will be described referring to Fig. 4. Fig. 4 is a sectional view showing a concept of the medical equipment treatment execution counter unit according to the second embodiment of the present invention. The same components herein as those of the first embodiment are designated as the same reference numerals, and explanations thereof, thus will be omitted. The first and the second signal lines 8 and 10 connected to the first and the second electric contacts 7 and 9, the battery 11, the counter unit 12 and the like are not shown.

A medical equipment treatment execution counter unit 1' according to the second embodiment of the present invention shown in Fig. 4 is different from the medical equipment treatment execution counter unit 1 according to the first embodiment in having a check valve 16 attached to the vent hole 15 of the housing 2.

The check valve 16 is formed of a cylindrical rod portion 17 with the outer diameter smaller than the inner diameter of the vent hole 15 such that the rod portion 17 is loosely fit therewith, and a coil spring 19 which urges the rod portion 17 downward shown in the drawing to the space 14 in the housing 2.

The rod portion 17 is formed of a first flange portion 18 provided to the lower end of the rod portion 17 which is loosely fitted with the vent hole 15 to protrude into the space 14 in the housing 2, a second flange portion 20 provided at the intermediate portion of the rod portion 17, which is positioned on the outer surface of the housing 2, and a knob portion 22 which extends upward from the second flange portion 20 of the rod member 17.

Each of the first flange portion 18 and the second flange portion 20 is formed into substantially a disc like shape having an outer diameter larger than the inner diameter of the vent hole 15. The coil spring 19 is interposed between the first flange portion 18 and the inner surface of the housing 2. The coil spring 19 urges the rod portion 17 toward the space 14 in the housing 2 such that the vent hole 15 is closed by the second flange portion 20. A doughnut-like packing 21 is arranged to the second flange portion 20 of the rod portion 17 on the housing 2 side. The packing 21 is tightly in contact with the second flange portion 20 to seal the vent hole 15. The knob portion 22 for the user to be operated is formed to extend upward from the second flange portion 20 of the rod portion 17.

The check valve 16 is provided such that the rod 17 is urged toward the space 14 in the housing 2 under the urging force of the coil spring 19 to allow the second flange portion 20 and the packing 21 to seal the vent hole 15.

The operation when the medical equipment which includes the medical equipment treatment execution counter unit 1' according to the second embodiment is subjected to the sterilization process through the autoclave sterilizing unit will be described.

In the preliminary vacuum step, when the chamber of the autoclave sterilizing unit is vacuumed, the pressure of the space 14 in the housing 2 becomes higher than the ambient pressure of the housing 2. When the pressure of the space 14 becomes higher than the ambient pressure of the housing 2, the coil spring 19 contracts to move the rod portion 17 upward in the drawing. When the rod portion 17 is moved upward in the drawing, the packing 21 moves away from the housing 2. Gas contained in the space 14 is sucked toward the periphery of the housing 2 via the vent hole 15. At this time, the elastic film 3 deforms in the expanding direction toward the space 14 at the lower pressure side (upward direction in the drawing). When the elastic film 3 is deformed to expand, the first electric contact 7 is moved upward in the drawing so as to be in contact with the second electric contact 9.

When the first electric contact 7 contacts the second electric contact 9, that is, they become ON state, the circuit including the battery 11 and the counter unit 12 is closed such that the power from the battery 11 is applied to the counter unit 12 via the first and the second electric contacts 7 and 9. The counter unit 12 detects the power application upon the contact between the first and the second electric contacts 7 and 9 to become ON, and counts the number of the power application.

When the pressure of the chamber, that is, the ambient pressure around the housing 2 is returned to the atmospheric pressure after the first vacuuming, as the pressure of the space 14 in the housing 2 is lower than the ambient pressure around the housing 2, gas around the housing 2 is likely to flow into the space 14. The urging force of the coil spring 19 and the pressure difference between the space 14 and the periphery of the housing 2 move the rod 17 downward in the drawing to allow the second flange portion 20 and the packing 21 to seal the vent hole 15. When the vent hole 15 is sealed, the pressure state of the space 14 resulting from the vacuuming is maintained. Accordingly, the elastic film 3 is kept deformed, thus maintaining ON state of the first and the second electric contacts 7 and 9 in contact with each other.

In the case where the second or subsequent vacuuming is performed in the chamber, the pressure of the chamber, that is, the ambient pressure around the housing 2 is substantially the same as that of the space 14. Accordingly, the vent hole 15 is kept sealed by the check valve 16. The elastic film 3, thus, is kept deformed, maintaining ON state of the first and the second electric contacts 7 and 9 in contact with each other.

Even after the entire sterilization process is finished, ON state of the first and the second electric contacts 7 and 9 is maintained in contact with each other. In the single sterilization process, the counter unit 12 detects to count the power application of only once resulting from ON state of the first and the second electric contacts 7 and 9 in contact with each other in the first vacuuming of the single sterilization process. The number of the power application resulting from ON state of the contact between the first and the second contacts 7 and 9, which has been counted by the counter unit 12 is displayed on the display unit 13.

After the entire sterilization process is finished, the user grasps the knob portion 22 at the upper end of the rod portion 17 to be pulled upward in the drawing in the atmospheric pressure to cause the pressure of the space 14 to be substantially the same as the atmospheric pressure. The elastic film 3 then returns to the original state before the sterilization process under the elastic force such that the first electric contact 7 moves away from the second electric contact 9 to become OFF state.

The medical equipment treatment execution counter unit 1' according to the second embodiment is structured to bring the first electric contact 7 into contact with the second electric contact 9 only once in the single sterilization process. The user is capable of recognizing that the accumulated number of the power application stored in the counter means 12 as the number of the actually performed sterilization process.

If the user does not pull the knob portion 22 of the check valve 16 upward after finishing the sterilization process, ON state of the first and the second electric contacts 7 and 9 is maintained in contact with each other to discharge the battery 11. In the case where the counter unit 12 detects the power application, the red LED and the like may be illuminated on the display unit 13 to alarm the operator of the sterilization process with respect to the operation of the knob portion 22. A portion similar to the knob portion 22 may be formed at the position of the medical equipment which is always contacted by the operator's hand such that the check valve 16 is semi-automatically operated upon touch of the operator for preparation or use of the medical equipment.

A medical equipment treatment execution counter unit according to the third embodiment of the present invention will be described referring to Fig. 5. Fig. 5 is a sectional view showing a concept of the medical equipment treatment execution counter unit according to the third embodiment of the present invention. The same components herein as those of the first embodiment are designated as the same reference numerals, and explanations thereof, thus will be omitted.

The medical equipment treatment execution counter unit according to the third embodiment shown in Fig. 5 serves to mechanically detect the number of vacuuming performed in the sterilization process.

A counter device 23 is disposed at the position opposite the elastic film 3 which is deformed by the pressure difference between the gas chamber 6 and the space 14. The counter device 23 is used for counting a plurality of numbers such that the number is counted up at every depression of a push button 24, and displaying the counted number. The counter device 23 is arranged to the housing 2 such that the push button 24 faces the substantially the center of the elastic film 3.

That is, when the elastic film 3 is deformed by the pressure difference between the gas chamber 6 and the space 14, the deformed elastic film 3 serves to depress the push button 24 of the counter device 23.

Thus, every time when the push button 24 of the counter device 23 is depressed by the elastic film 3 caused by the pressure difference between the gas chamber 6 and the space 14 in the vacuuming of the sterilization process, the number is counted up. The user divides the number displayed on the counter device 23 by the number of vacuuming performed in the single sterilization process as one unit to recognize the accumulated number of the sterilization process executed to the medical equipment. The medical equipment treatment execution counter unit according to the third embodiment may be operated over an elongated service life as no battery is required.

In the embodiment, the counter device 23 is provided at the position opposite the elastic film 3 in the housing 2. However, the medical equipment treatment execution counter device may be formed into an arbitrary structure so long as the pressure force generated by deformation of the elastic film 3 is mechanically transmitted to the counter unit. For example, a pressure force transmitting member, for example, wire, link mechanism or the like may be disposed at the position opposite the elastic film 3 in the housing 2 such that the pressure force generated by the elastic film 3 is transmitted to the counter device 23 disposed on the outer portion of the housing 2 by means of the transmitting member.

That is, deformation of the elastic film 3 depresses the push button 24 of the counter device 23 via the transmitting member such that the deformation of the elastic film 3 is counted by the counter device 23.

### Industrial Applicability

The medical equipment and the medical equipment treatment execution counter unit according to the present invention allow the user to obtain the number of the sterilization process executed to the medical equipment objectively, and to subject the medical equipment to the maintenance/repair recommended by the manufacturer before the number of the sterilization allowable to the medical equipment is reached. This makes it possible to use the medical equipment safely for an elongated service life.

## Claims

1. A medical equipment provided with a count unit for counting a number of executing a treatment including at least one of washing, disinfection and sterilization to the medical equipment, wherein the count unit includes:
a sealed chamber which has an elastic film that exhibits elasticity at least partially and a sealed space isolated from the outside by the elastic film; and
count means for counting the number of deformation of the elastic film.

2. A medical equipment according to Claim 1, wherein the sealed chamber is provided in a space of a housing contained therein having a vent hole formed to communicate the space with the outside.

3. A medical equipment according to Claim 1 or 2, wherein the housing is provided to a part of an exterior member of the medical equipment or formed as a part of the exterior member.

4. A medical equipment according to Claim 2 or 3, further comprising a check valve for sealing the vent hole when a pressure of the space in the housing becomes negative with respect to the pressure outside the housing.

5. A medical equipment according to any one of Claims 1 to 4, wherein the count unit further includes a display unit which displays a value counted by the count means.

6. A medical equipment according to any one of Claims 1 to 5, wherein the count means includes a first electric contact disposed on the elastic film, a second electric contact disposed on a position opposite the first electric contact, and a counter unit which detects a power application state where the first electric contact and the second electric contact are brought into contact by deformation of the elastic film, and counts to store the number of the power application.

7. A medical equipment according to any one of Claims 1 to 5, wherein the count means includes a push button mechanically depressed by the deformed elastic film, and a counter device which counts up a number of depression of the push button.

8. A medical equipment treatment execution counter unit which counts a number of executing a treatment including at least one of washing, disinfection and sterilization to the medical equipment, comprising:
a sealed chamber which has an elastic film that exhibits elasticity at least partially, and a sealed space isolated from the outside by the elastic film; and
count means for counting the number of deformation of the elastic film.
